# EUROPEAN PATENT APPLICATION

(11) **EP 4 733 310 A1**
(43) Date of publication of application: **29.04.2026**
(21) Application number: 24826301.4
(22) Date of filing: 21.06.2024
(51) Int. Cl.: C07K 7/08, A61P 25/00, A61K 38/00

(54) **NOVEL PEPTIDE AND USE THEREOF**

(30) Priority: 23.06.2023 KR 20230080938
(71) Applicant: Gemvax & Kael Co., Ltd., Daejeon 34036 (KR)
(72) Inventor: KIM, Sang Jae, Seoul 06360 (KR)
(74) Representative: Stolmár & Partner Patentanwälte PartG mbB
(86) International application number: PCT/KR2024/008597
(87) International publication number: WO 2024/262986

(57) **Abstract**

The present invention relates to a novel peptide that exhibits preventive, alleviating, or therapeutic efficacy against amyloidosis and/or macular degeneration, is safe in vivo, and has minimal side effects, including adverse reactions, as well as a pharmaceutical composition and a health functional food including the peptide.

## Description

### CROSS-REFERENCE TO RELATED APPLICATION AND CLAIM OF PRIORITY

This application claims priority to Korean Patent Application No. 10-2023-0080938 filed on June 23, 2023 in the Korean Intellectual Property Office (KIPO), the entire disclosure of which is incorporated by reference herein.

### [Technical Field]

The present invention relates to a novel peptide and the use thereof.

### [Background Art]

Amyloidosis is a general term for a group of disorders in which abnormal proteins known as amyloid fibrils accumulate in tissues.

Alzheimer's disease (AD) is the most common type of amyloidosis in humans. Alzheimer's disease is also the most common degenerative brain disease associated with dementia, and may be accompanied by declines in memory, language ability, and spatial perception ability, as well as personality impairment and behavioral changes.

Pathologically, Alzheimer's disease is characterized by the infiltration of amyloid-β plaques, composed of abnormal amyloid proteins, apoptotic neurons, and inflammatory cells, into the extracellular space of neurons, along with gliosis. Various inflammatory processes and cytokines also play a key role in the pathogenesis of Alzheimer's disease. Inflammatory cytokines may be secondary to tissue damage in Alzheimer's disease or serve as indicators of immune responses.

The prevalence of Alzheimer's disease is increasing due to aging, and the resulting socioeconomic costs are also increasing.

However, to date, no treatment has been developed to completely cure Alzheimer's disease, and only drugs, such as AChE inhibitors and NMDA receptor antagonists, known to delay the rate of memory decline, are currently used as treatments.

Age-related macular degeneration (AMD) is an ocular disease in which the function of the macula, the central part of the retina where images are formed, is impaired, resulting in gradual loss of vision starting from the center of the visual field and eventually leading to blindness. AMD can be classified into dry AMD and wet AMD.

Dry AMD occurs when retinal cells are continuously exposed to stress conditions such as oxidative stress, leading to retinal cell death and degeneration. Because dry AMD may progress to wet AMD, treatment at the dry AMD stage is particularly important.

In the case of wet AMD, neovascularization induced by vascular endothelial growth factor (VEGF) is known to be a direct cause, and drugs targeting VEGF, such as Eylea^{®}, are currently used as therapeutic agents.

Unlike wet AMD, for which many therapeutic agents have been commercialized, only one FDA-approved drug exists for dry AMD to date. Therefore, the development of a therapeutic agent for dry AMD capable of controlling the early progression of AMD is urgently required.

Meanwhile, it was reported that a 16-amino-acid peptide GV1001, selected from the reverse transcriptase of human telomerase (hTERT), has anticancer, anti-inflammatory, and anti-oxidative efficacies, and is also efficacious in the treating Alzheimer's disease. Further, it was confirmed that GV1001 has completed toxicity tests in a number of clinical trials targeting different diseases, and exhibits stability with respect to side effects.

However, despite its excellent efficacy, GV1001 is readily cleaved in vivo and has a short half-life, which necessitates frequent administration when used as a drug.

Accordingly, as a result of intensive studies to improve the stability of the GV1001 peptide, the present inventors have developed a novel peptide that not only exhibits enhanced stability but also provides superior therapeutic effects for diseases such as amyloidosis and macular degeneration.

### [Summary of Invention]

### [Problems to be Solved by Invention]

An object of the present invention is to provide a novel peptide that exhibits efficacy for preventing or treating amyloidosis and macular degeneration.

Another object of the present invention is to provide a pharmaceutical composition for preventing or treating Alzheimer's disease.

Yet another object of the present invention is to provide a health functional food for preventing or alleviating Alzheimer's disease.

Still another object of the present invention is to provide a pharmaceutical composition for preventing or treating cerebral amyloid angiopathy (CAA).

In addition, another object of the present invention is to provide a health functional food for preventing or alleviating cerebral amyloid angiopathy.

Further, another object of the present invention is to provide a pharmaceutical composition for preventing or treating age-related macular degeneration.

Furthermore, another object of the present invention is to provide a health functional food for preventing or alleviating age-related macular degeneration.

### [Means for Solving Problems]

1. A peptide of Formula 1 below or a pharmaceutically acceptable salt thereof: 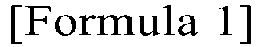 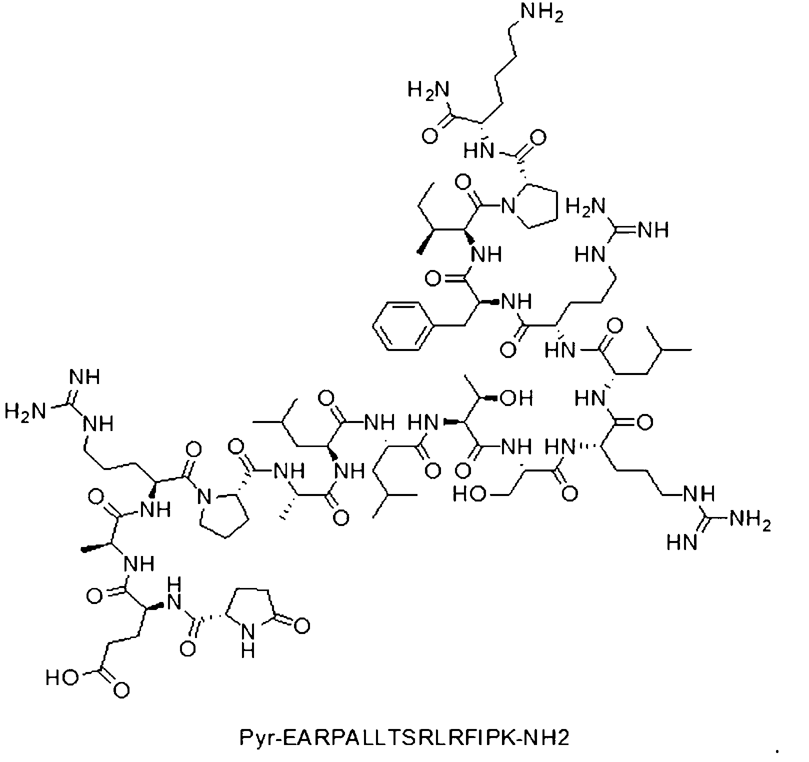
2. A pharmaceutical composition for preventing or treating amyloidosis, comprising the peptide of the above 1 or a pharmaceutically acceptable salt thereof.
3. The pharmaceutical composition according to the above 2, wherein the amyloidosis is Alzheimer's disease or cerebral amyloid angiopathy (CAA).
4. The pharmaceutical composition according to the above 2, wherein the prevention or treatment of amyloidosis is achieved by reducing the deposition of amyloid-β (Aβ).
5. A pharmaceutical composition for preventing or treating macular degeneration, comprising the peptide of the above 1 or a pharmaceutically acceptable salt thereof.
6. The pharmaceutical composition according to the above 5, wherein the macular degeneration is age-related macular degeneration (AMD).
7. The pharmaceutical composition according to the above 5, wherein the prevention or treatment of macular degeneration is achieved by reducing the degeneration of retinal pigment epithelial cells (RPE cells).
8. A health functional food for preventing or alleviating amyloidosis, comprising the peptide of Formula 1 below or a pharmaceutically acceptable salt thereof: 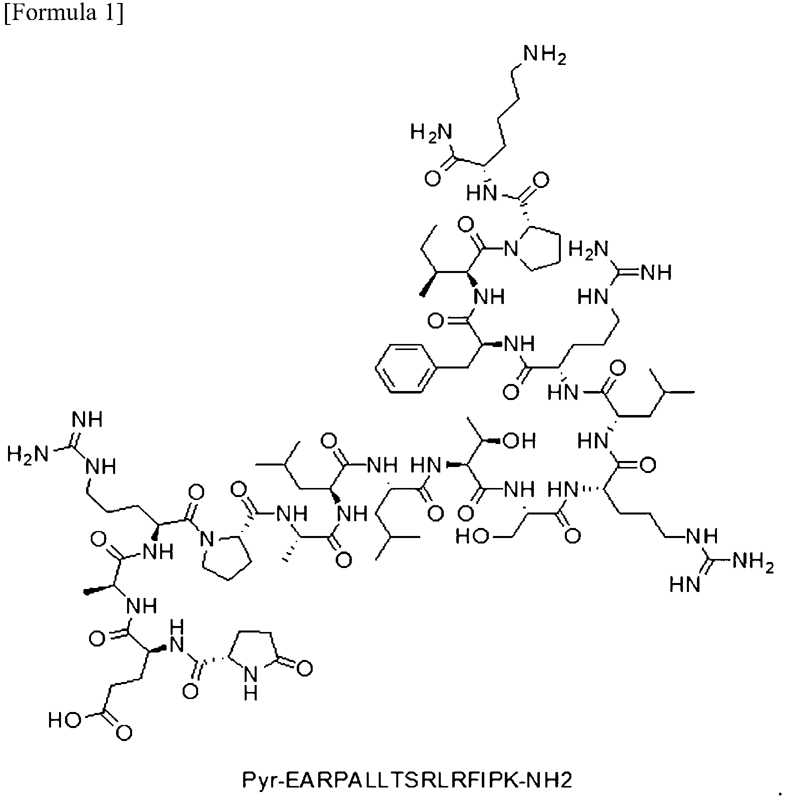
9. The health functional food according to the above 8, wherein the amyloidosis is Alzheimer's disease or cerebral amyloid angiopathy.
10. The health functional food according to the above 8, wherein the prevention or alleviation of amyloidosis is achieved by reducing the deposition of amyloid-β (Aβ).
11. A health functional food for preventing or alleviating macular degeneration, comprising a peptide of Formula 1 below or a food-acceptable salt thereof: 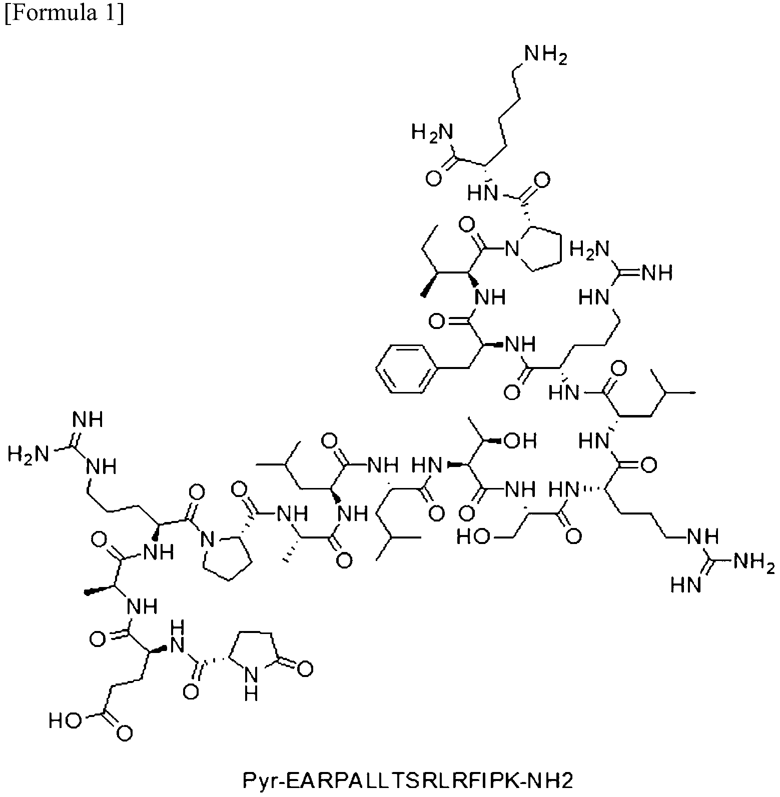
12. The health functional food according to the above 11, wherein the macular degeneration is age-related macular degeneration.
13. The health functional food according to the above 11, wherein the prevention or alleviation of macular degeneration is achieved by reducing the degeneration of retinal pigment epithelial cells.

### [Advantageous effects]

The peptide of Formula 1 of the present invention and the pharmaceutically acceptable salt thereof exhibit excellent amyloid-β plaque deposition-reducing effects.

The peptide of Formula 1 of the present invention and the pharmaceutically acceptable salt thereof exhibit amyloid-β protein level-reducing effects.

The peptide of Formula 1 of the present invention and the pharmaceutically acceptable salt thereof exhibit inflammatory cytokine production-reducing effects.

The peptide of Formula 1 of the present invention and the pharmaceutically acceptable salt thereof exhibit gliosis-reducing effects.

The peptide of Formula 1 of the present invention and the pharmaceutically acceptable salt thereof exhibit reactive oxygen species (ROS)-reducing effects.

The peptide of Formula 1 of the present invention and the pharmaceutically acceptable salt thereof exhibit retinal pigment epithelial cell degeneration-reducing effects.

The pharmaceutical composition and health functional food including the peptide of Formula 1 of the present invention or the salt thereof may exhibit preventive, alleviating, and/or therapeutic effects for Alzheimer's disease, cerebral amyloid angiopathy, and age-related macular degeneration.

The peptide, pharmaceutical composition, and health functional food of the present invention may exhibit stable, long-lasting effects in vivo.

The peptide of the present invention is more stable than GV1001 and exhibits superior therapeutic efficacy for amyloidosis and macular degeneration.

The peptide of the present invention may exhibit therapeutic effects for amyloidosis or macular degeneration even with lower doses or fewer administrations compared to GV1001.

### [Brief Description of Drawings]

FIG. 1 illustrates the results of an experiment confirming the stability of GV2001 and GV1001 in human plasma.
FIG. 2 illustrates the results of confirming the pharmacokinetic (PK) profiles of GV2001 and GV1001 administered intravenously to rats.
FIG. 3 illustrates the results of confirming the pharmacokinetic profiles of GV2001 and GV1001 administered subcutaneously to rats.
FIG. 4 illustrates the results of an experiment evaluating the alleviation of impairment in spatial learning ability in a mouse model of Alzheimer's disease.
FIGS. 5 and 6 illustrate the results of experiments evaluating the amyloid-β-reducing efficacy of GV2001 using immunoblotting and ELISA.
FIG. 7 illustrates the results of an experiment evaluating the inflammatory cytokine-reducing efficacy of GV2001.
FIGS. 8 to 11 illustrate the results of experiments evaluating the amyloid-β plaque-reducing and amyloid-β-reducing efficacy of GV2001 through histological analysis.
FIGS. 12 and 13 illustrate the results of experiments evaluating the gliosis-reducing efficacy of GV2001.
FIG. 14 illustrates the results of experiments evaluating the reactive oxygen species-reducing efficacy of GV2001.
FIG. 15 illustrates the results of experiments evaluating the retinal pigment epithelial cell degeneration-reducing efficacy of GV2001.

### [Mode for Carrying out Invention]

The present invention provides a peptide of Formula 1 below or a pharmaceutically acceptable salt thereof:

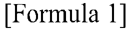

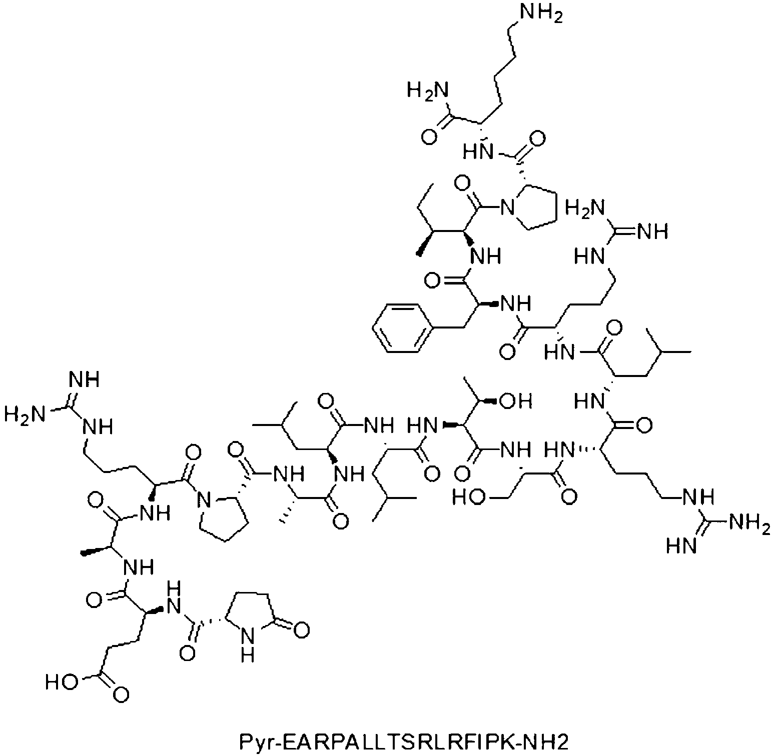

In the present invention, the peptide of Formula 1 includes functional equivalents thereof. The term "functional equivalent" refers to a peptide exhibiting substantially the same physiological activity as the peptide of Formula 1.

The present invention provides a pharmaceutical composition for preventing or treating amyloidosis, including a peptide of Formula 1 or a pharmaceutically acceptable salt thereof as an active ingredient.

As used herein, the term "pharmaceutically acceptable" refers to a property that is non-toxic to cells or organisms exposed to the composition.

As used herein, the term "amyloidosis" collectively refers to diseases caused by amyloid-β deposition, including Alzheimer's disease, cerebral amyloid angiopathy, and Down syndrome.

In one embodiment, amyloidosis may be Alzheimer's disease or cerebral amyloid angiopathy.

As used herein, the term "prevention of amyloidosis" refers to any action that inhibits, delays, or reduces the onset or progression of amyloidosis.

As used herein, the term "alleviation of amyloidosis" and "treatment of amyloidosis" refer to any action that alleviates or beneficially modifies the symptoms of a subject who has developed, or is at risk of developing, amyloidosis.

In one embodiment, the prevention, alleviation, and/or treatment of amyloidosis may be achieved by reducing amyloid-β.

In one embodiment, the prevention, alleviation, and/or treatment of amyloidosis may be achieved by reducing amyloid-β plaques.

In one embodiment, the prevention, alleviation, and/or treatment of amyloidosis may be achieved by reducing inflammatory cytokines.

In one embodiment, the prevention, alleviation, and/or treatment of amyloidosis may be achieved by reducing gliosis.

In one embodiment, the prevention, alleviation, and/or treatment of amyloidosis may be achieved by alleviating impairment in spatial learning ability of a subject who has developed, or is at risk of developing, amyloidosis.

The present invention provides a pharmaceutical composition for preventing or treating macular degeneration, including a peptide of Formula 1 or a pharmaceutically acceptable salt thereof as an active ingredient.

In the present invention, the "macular degeneration" includes senile macular degeneration, age-related macular degeneration (AMD), macular degeneration in the elderly, myopic macular degeneration (MMD), and macular dystrophy.

In the present invention, the "age-related macular degeneration" includes dry macular degeneration and wet macular degeneration.

In one embodiment, the macular degeneration may be senile macular degeneration.

In one embodiment, the macular degeneration may be dry macular degeneration.

In the present invention, the "prevention of macular degeneration" refers to any action that inhibits, delays, or reduces the onset or progression of macular degeneration.

In the present invention, the "alleviation of macular degeneration" and "treatment of macular degeneration" mean any action that alleviates or beneficially modifies the symptoms of a subject who has developed, or is at risk of developing, macular degeneration.

In one embodiment, the prevention, alleviation, and/or treatment of macular degeneration may be achieved by reducing reactive oxygen species.

In one embodiment, the prevention, alleviation, and/or treatment of macular degeneration may be achieved by reducing the degeneration of retinal pigment epithelial cells.

The pharmaceutical composition of the present invention may include the active ingredient alone or may further include one or more pharmaceutically acceptable carriers, excipients, or diluents.

The carrier, excipient, or diluent that may be included in the pharmaceutical composition of the present invention may include lactose, dextrose, sucrose, dextrin, maltodextrin, sorbitol, mannitol, xylitol, erythritol, maltitol, starch, gum acacia, alginate, gelatin, calcium phosphate, calcium silicate, cellulose, methyl cellulose, microcrystalline cellulose, polyvinylpyrrolidone, water, methyl hydroxybenzoate, propyl hydroxybenzoate, talc, magnesium stearate, or mineral oil, but is not limited thereto.

The pharmaceutical composition of the present invention may be administered to a subject.

As used herein, the term "administration" refers to introducing a predetermined substance into a subject by an appropriate method, and the term "subject" refers to all animals, such as livestock and mice, and may be mammals, including humans, for example.

The administration route of the pharmaceutical composition of the present invention may be oral, intravenous, intramuscular, intra-arterial, intramedullary, intrathecal, intracardiac, transdermal, subcutaneous, intraperitoneal, intranasal, intestinal, topical, sublingual, or rectal, but is not limited thereto.

In one embodiment, the composition of the present invention may be administered orally or parenterally.

When the composition of the present invention is administered parenterally, it is preferable to select an administration method such as topical application, intraperitoneal injection, intrarectal injection, subcutaneous injection, intravenous injection, intramuscular injection, or intrathoracic injection, but is not limited thereto.

The pharmaceutical composition of the present invention may be a solid preparation for oral administration, such as a tablet, pill, powder, granule or capsule.

The pharmaceutical composition of the present invention may be a liquid preparation for oral administration, such as a suspension, a solution, an emulsion or a syrup.

The pharmaceutical composition of the present invention may be a preparation for parenteral administration, such as a sterile aqueous solution, a non-aqueous solution, a suspension, an emulsion, a lyophilized preparation or a suppository.

The pharmaceutical composition of the present invention may be administered to a subject in a pharmaceutically effective amount, and the term "pharmaceutically effective amount" refers to an amount which is sufficient to provide a desired pharmacological effect at a reasonable benefit/risk ratio applicable to medical treatment.

The pharmaceutically effective amount of the pharmaceutical composition of the present invention may be determined based on factors including the severity of the disease, the activity of the pharmaceutical composition, the sensitivity of the subject or patient to the pharmaceutical composition, the time of administration, the route of administration, the excretion rate, the duration of treatment, and concomitant medications, as well as other factors well known in the medical field, and may be appropriately selected by those skilled in the art.

The pharmaceutical composition of the present invention may be administered as a standalone therapeutic agent or in combination with other conventional therapeutic agents. When administered in combination, it may be administered sequentially or concurrently, and may be administered as a single dose or in multiple doses, and this may be easily determined by those skilled in the art.

The present invention provides a health functional food for preventing or alleviating amyloidosis, including a peptide of Formula 1 or a food-acceptable salt thereof.

The present invention also provides a health functional food for preventing or alleviating macular degeneration, including a peptide of Formula 1 or a food-acceptable salt thereof.

The health functional food of the present invention refers to a food manufactured and processed using raw materials or ingredients having beneficial functionality for the human body, as defined by the Health Functional Food Act of Korea. The term "functionality" refers to ingestion for the purpose of obtaining beneficial effects for health use such as controlling nutrients related to the structure or function of the human body or physiological actions.

The health functional food of the present invention may include conventional food additives, and the suitability of such food additives shall be determined on the basis of the standards and specifications of the corresponding items according to the General Regulations and General Test Methods of the Food Additives Code approved by the Korean Ministry of Food and Drug Safety, unless otherwise specified.

The items listed in the Food Additives Code may include, for example: chemical synthetic substances such as ketones, glycine, potassium citrate, nicotinic acid, and cinnamic acid; natural additives such as gardenia blue pigment, licorice extract, crystalline cellulose, red yeast rice pigment, and guar gum; and mixed preparations such as L-glutamate sodium preparations, alkaline agents for noodles, preservative preparations, and tar color preparations, and the like.

The health functional food of the present invention may include a peptide of Formula 1 in an amount of 0.01 to 95% by weight, preferably 1 to 80% by weight, based on the total weight of the health functional food, for the purpose of preventing and/or alleviating disease. In addition, the health functional food may be manufactured and processed into tablets, capsules, powders, granules, liquids, pills, or the like, for the purpose of preventing and/or alleviating disease.

Hereinafter, the present invention will be described in detail by way of examples to further illustrate the present invention. However, the following examples are provided only to facilitate understanding of the present invention, and the scope of the present invention is not limited thereto.

### Examples

### [Example 1] Preparation of GV2001

A peptide composed of 17 amino acids (hereinafter referred to as GV2001), represented by the structural formula of Formula 1 below, was prepared.

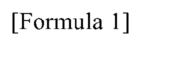

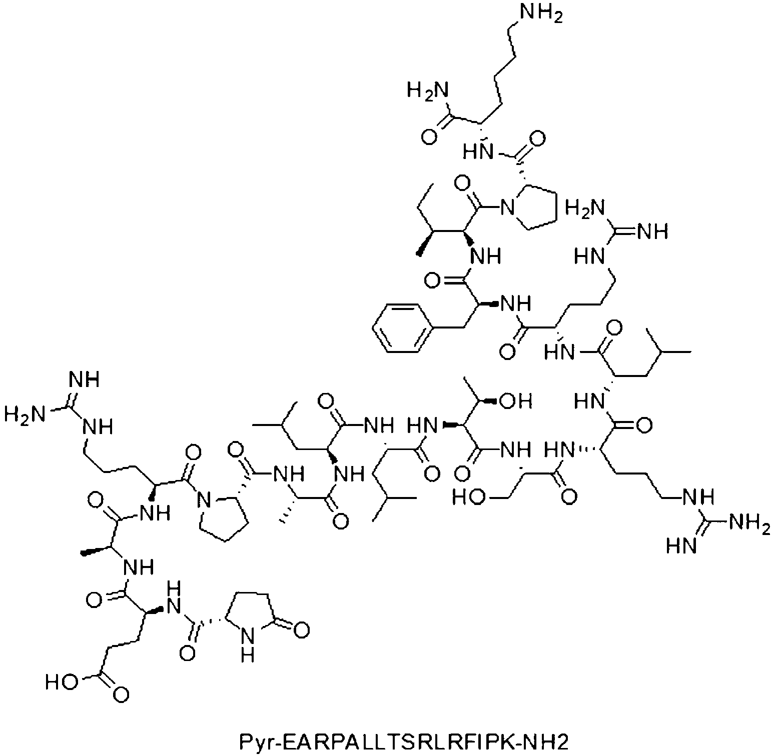

GV2001 was synthesized by sequentially coupling amino acids from the C-terminus using the Fmoc solid-phase peptide synthesis (SPPS) method known in the art.

All amino acid starting materials used for peptide synthesis were protected at the N-terminus with Fmoc or Boc, and all residues were protected with Boc, t-Bu (t-butylester), or Pbf (2,2,4,6,7-pentamethyl dihydro-benzofuran-5-sulfonyl), etc., which are removable under acidic conditions. For example:

Fmoc-Ala-OH, Fmoc-Arg(Pbf)-OH, Boc-Pyr-OH, Fmoc-Lys(Boc)-OH, Fmoc-Pro-OH, Fmoc-Leu-OH, Fmoc-Ile-OH, Fmoc-Phe-OH, Fmoc-Ser-OH, Fmoc-Thr-OH, Fmoc-Lys(Boc)-OH, Fmoc-Arg(Pbf)-OH.

As coupling reagents, diisopropylcarbodiimide (DIC), dimethylformamide (DMF), ethyl(hydroxyamino)cyanoacetate (Oxyma), and isopropyl alcohol (IPA) were used. Piperidine in DMF (20%) was used for Fmoc removal. A cleavage cocktail [95% trifluoroacetic acid (TFA)/5% water (H₂O)/50 mg/mL dithiothreitol (DTT)] was used to cleave the synthesized peptide from the resin and remove the protecting groups.

The peptide was synthesized in a state in which the starting amino acid, bearing a protecting group, was first bound to a solid support, by sequentially coupling each subsequent amino acid, washing with a solvent, and removing the protecting group in a repeated manner. The synthesized peptide was cleaved from the resin, purified by HPLC, confirmed by MS, and lyophilized.

For the counter ion exchange process, GV2001, dissolved in water in the form of an HOAc salt, was converted into a chloride form using AmberChrom ion-exchange resin. The chloride-exchanged GV2001 was filtered through a PVDF filter and lyophilized.

The specific synthesis process for GV2001 is as follows:

### (1) Coupling

Amino acids were coupled sequentially from the C-terminus to the N-terminus to the Fmoc-Rink methylbenzhydrylamine (MBHA) resin by adding each amino acid together with the coupling reagents DIC/Oxyma/IPA dissolved in DMF. The resin was then washed with DMF.

### (2) Fmoc and Boc Deprotection

Deprotection was performed by adding piperidine in DMF (20%) to remove Fmoc and Boc protecting groups.

### (3) Cleavage

After completion of the peptide synthesis, the peptide was cleaved from the resin by treating the synthesized peptide-resin with a cleavage cocktail.

### (4) Filtration

The resulting mixture was concentrated, precipitated with MTBE/hexane, filtered, and dried.

### (5) Purification

The resulting dried filtrate was purified by preparative HPLC (Prep-HPLC), the molecular weight was confirmed by LC/MS, and the purified peptide was lyophilized.

### (6) Preparation of Powder

The lyophilized peptide was converted into a chloride form using AmberChrom ion-exchange resin, filtered through a PVDF filter, and lyophilized again to obtain the peptide in powder form.

### [Example 2] Confirmation of GV2001 Stability in Human Plasma

GV2001 and GV1001 were each added to human plasma and diluted to a concentration of 5 µM, followed by incubation at 37 °C for 10, 60, and 240 minutes with stirring. The reaction mixture was collected and pretreated by plasma protein precipitation using methanol containing 0.1% formic acid. The resulting supernatants were collected and analyzed by LC-MS under the conditions shown in Table 1 below to determine the percentage of residual peptide relative to the initial peptide amount.

**[TABLE 1]**

| | | | | |
|---|---|---|---|---|
| | Column | ACQUITY UPLC^{®} BEH C₁₈ Column (2.10 mm × 50 mm, 1.7 µm) | | |
| | Column temperature | 35°C | | |
| | Mobile phase | Time (min) | A: 0.1% Formic acid in Water | B: 0.1% Formic acid in Acetonitrile |
| | | 0.00 | 95% | 5% |
| | | 0.02 | 95% | 5% |
| HPLC conditions | | 2.80 | 5% | 95% |
| | | 3.20 | 5% | 95% |
| | | 3.50 | 95% | 5% |
| | | 5.00 | 95% | 5% |
| | Flow rate | 0.4 mL/min | | |
| | Dose volume | 2 µL | | |
| MS conditions | Ion source | ES + Source | | |
| | Capillary (kV) | 1.00 | | |
| | Desolvation Temperature | 500°C | | |
| | Gas flow | - Desolvation (L/hr): 800 | | |
| | | - Con (L/hr): 150 | | |
| | Nebulizer | 7.0 bar | | |

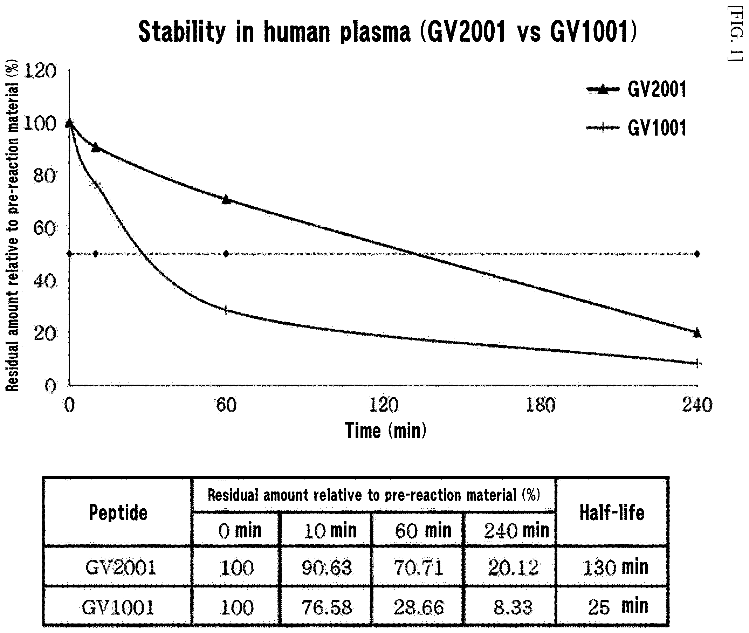

As shown in FIG. 1, the percentage of residual peptide relative to the peptide amount before reaction for GV2001 was higher at each time point than that of GV1001. This confirms that GV2001 exhibits greater stability in plasma compared to GV1001.

### [Example 3] Pharmacokinetic Profile of GV2001

To determine the pharmacokinetic profile of GV2001 prepared in Example 1, plasma was collected from rats administered with GV2001. The collected plasma was pretreated by protein precipitation using nine times the volume of methanol containing 0.1% formic acid, followed by centrifugation at 16,100 g for 10 minutes. The resulting supernatant was collected and subjected to LC-MS analysis under the conditions shown in Table 2.

**[TABLE 2]**

| | | | | |
|---|---|---|---|---|
| | Column | ACQUITY UPLC^{•} BEH C18 Column (2.10 mm x 50 mm, 1.7 µm) | | |
| | Column temperature | 35°C | | |
| | Mobile phase | Time (min) | A: 0.1% Formic acid in Water | B: 0.1% Formic acid in Acetonitrile |
| | | 0.00 | 95% | 5% |
| | | 0.02 | 95% | 5% |
| HPLC conditions | | 0.07 | 5% | 95% |
| | | 1.70 | 5% | 95% |
| | | 1.75 | 95% | 5% |
| | | 3.00 | 95% | 5% |
| | Flow rate | 0.5 mL/min | | |
| | Time (min) | 2 µL | | |
| MS conditions | Ion source | ES + Source | | |
| | Capillary (kV) | 1.00 | | |
| | Desolvation Temperature | 500°C | | |
| | Gas flow | - Desolvation (L/hr): 800 | | |
| | | - Con (L/hr): 150 | | |
| | Nebulizer | 7.0 bar | | |

### 3-1. Pharmacokinetic Profile of GV2001 Through Intravenous Administration

GV2001 and GV1001 were administered intravenously (IV) to three Sprague Dawley rats in each group. Blood samples were collected at 0.033, 0.083, 0.17, 0.25, 0.5, 1, and 2 hours after administration. The collected blood samples were centrifuged to separate plasma, pretreated, and analyzed using LC-MS. Pharmacokinetic parameters were calculated using the non-compartmental analysis model of the Phoenix WinNonlin (Pharsight, ver. 6.4, USA) program.

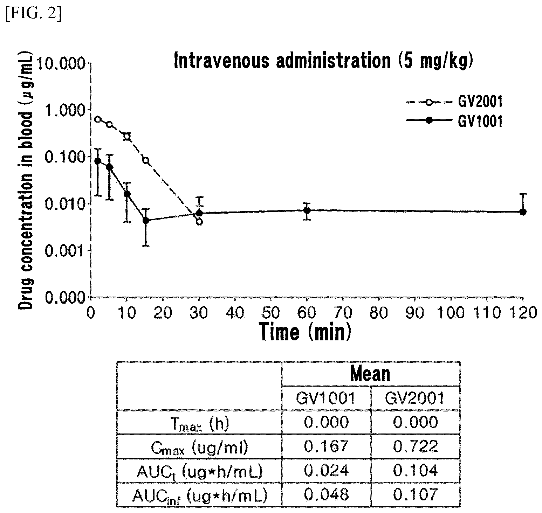

As shown in FIG. 2, the AUCt value of GV2001 was 0.107 µg·h/mL, which was more than four times higher than that of GV1001 (0.024 µg·h/mL). This confirms that GV2001 exhibits greater in vivo stability than GV1001 and remains in the body for a longer period without degradation.

### 3-2. Pharmacokinetic Profile of GV2001 Through Subcutaneous Administration

GV2001 and GV1001 were administered subcutaneously (SC) to three Sprague Dawley rats in each group. Blood samples were collected at 0.033, 0.083, 0.17, 0.25, 0.5, 1, and 2 hours after administration. The collected blood samples were centrifuged to separate plasma, pretreated, and analyzed using LC-MS. Pharmacokinetic parameters were calculated using the non-compartmental analysis model of Phoenix WinNonlin (Pharsight ver. 6.4, USA).

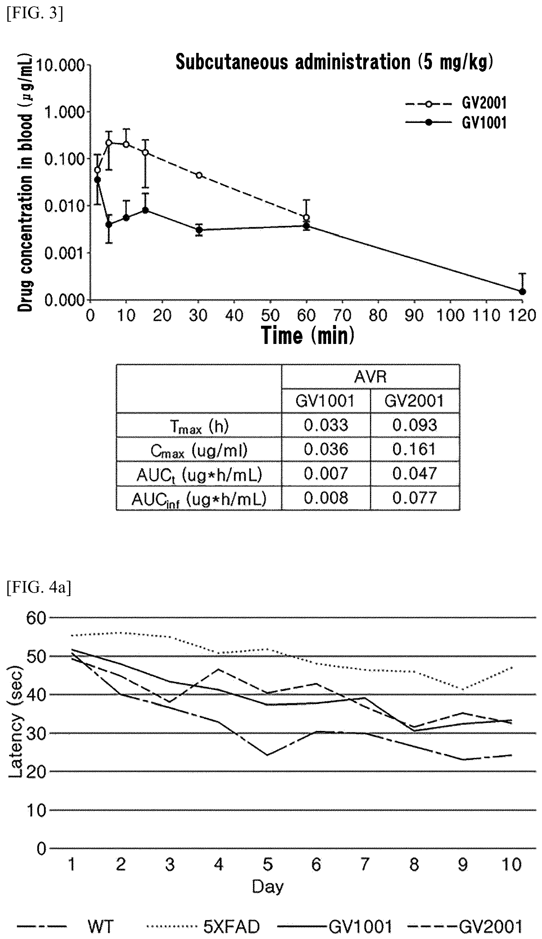

As shown in FIG. 3, GV2001 exhibited greater in vivo stability than GV1001, remaining in the body for a longer period without degradation following subcutaneous administration.

### [Example 4] GV2001 Efficacy Evaluation Based on an Alzheimer's Disease Animal Model

An efficacy evaluation of GV2001 based on an Alzheimer's disease animal model was conducted according to Table 3 below.

**[TABLE 3]**

| Group | Number of animals | Administered article | Dose volume | Administration frequency | Administration period | Route of administration |
|---|---|---|---|---|---|---|
| WT | 11 | Saline | 100 µl | 3 times/week | 8 weeks | Subcutaneous injection |
| 5xFAD | 10 | Saline | 100 µl | | | |
| GV1001 | 10 | GV1001 | 2 mg/kg, 100 µl | | | |
| GV2001 | 10 | GV2001 | 2 mg/kg, 100 µl | | | |

5xFAD mice are a well-established mouse model of Alzheimer's disease, in which amyloid-β is produced in neurons. Amyloid-β accumulation begins at approximately 2 months of age, neuronal damage progresses from around 6 months of age, and impairment in spatial learning ability develops from approximately 9 months of age. The mice were purchased from Jackson Laboratory, and seven-month-old mice obtained through breeding were used in the experiment.

### 4-1. Evaluation of the alleviation of impairment in spatial learning ability through the Morris Water Maze Test

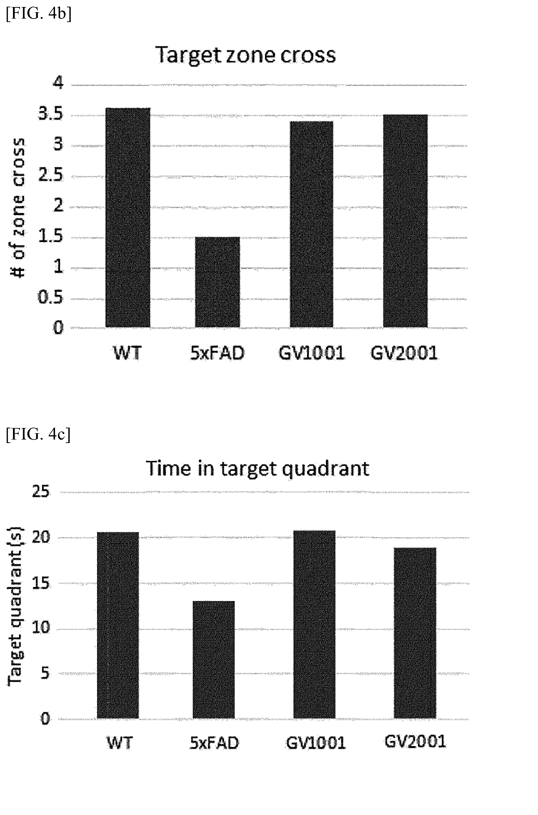

Water maze test results showed that the WT group gradually located the hidden platform faster over the 10-day training period, exhibiting a clear difference from the 5xFAD group. The 5xFAD group did not show a substantial reduction in latency during the 10-day training period; however, the GV1001 and GV2001 groups demonstrated marked improvements in latency (FIG. 4A). On day 11, a probe test was performed. Zone-cross analysis revealed that the number of platform-zone crossings was reduced in the 5xFAD group compared with the WT group, whereas the GV1001 and GV2001 groups exhibited improvements (FIG. 4B). In addition, the time spent in the target quadrant was increased in the GV1001 and GV2001 groups, confirming improvements in spatial learning ability (FIG. 4C).

### 4-2. Evaluation of Amyloid-β Reduction Through Immunoblot and ELISA

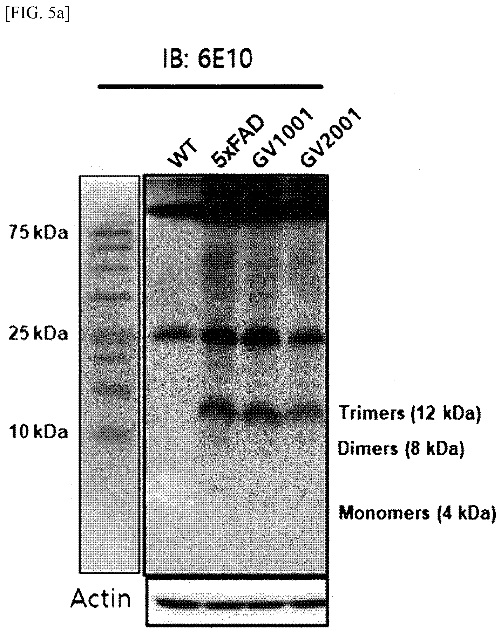

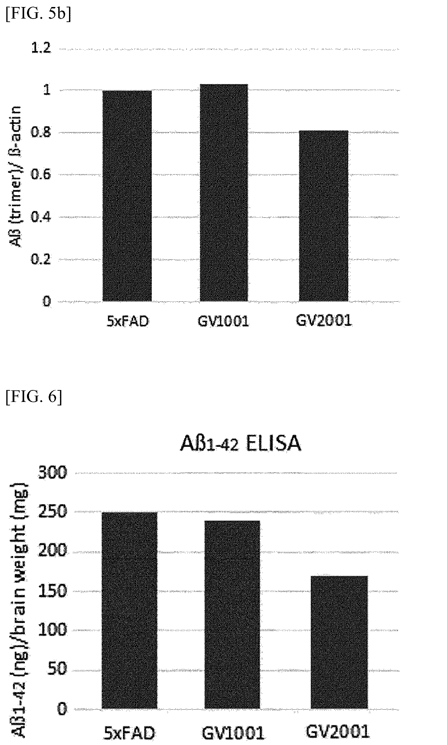

In order to determine amyloid-β levels in the brains of each group of mice, immunoblotting was performed on mouse hippocampal protein samples using an anti-amyloid antibody (6E10). The results showed a reduction in the GV2001 group (FIG. 5). Furthermore, amyloid-β levels in mouse cerebral cortex protein samples were measured using an Aβ1-42 ELISA, and the results showed a reduced level of amyloid-β protein in the GV2001 group (FIG. 6).

### 4-3. Evaluation of Inflammatory Cytokine Reduction

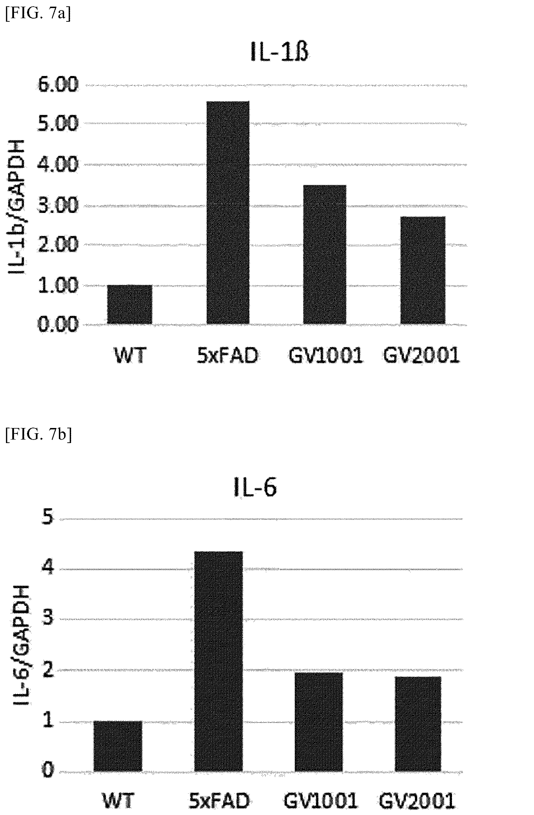

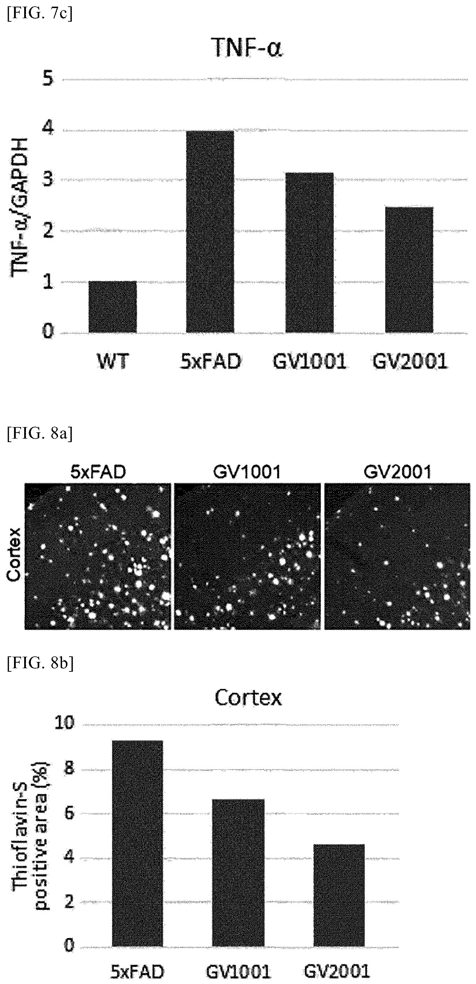

mRNA levels of cytokines involved in inflammation (IL-1β, IL-6, and TNF-α) were analyzed using qRT-PCR in mouse hippocampal samples. The 5xFAD group showed elevated cytokine expression compared to the WT group for all cytokines, while the GV2001 group showed a greater reduction in cytokine expression compared to the 5xFAD group (FIG. 7).

### 4-4. Evaluation of Amyloid-β Plaques and Amyloid-P Reduction Through Histological Analysis

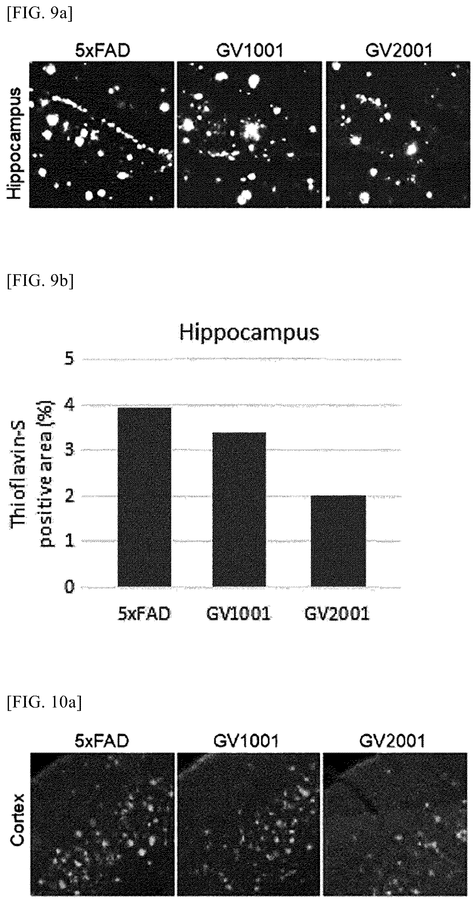

Mouse brain tissue sections were stained with thioflavin-S and anti-amyloid antibody (6E10) to identify amyloid-β plaques and amyloid-β protein, respectively. Thioflavin-S staining results revealed a reduction in amyloid-β plaque deposition in the cerebral cortex in both the GV1001 and GV2001 groups (FIG. 8). In the hippocampus, the GV2001 group showed substantially reduced plaque deposition (FIG. 9).

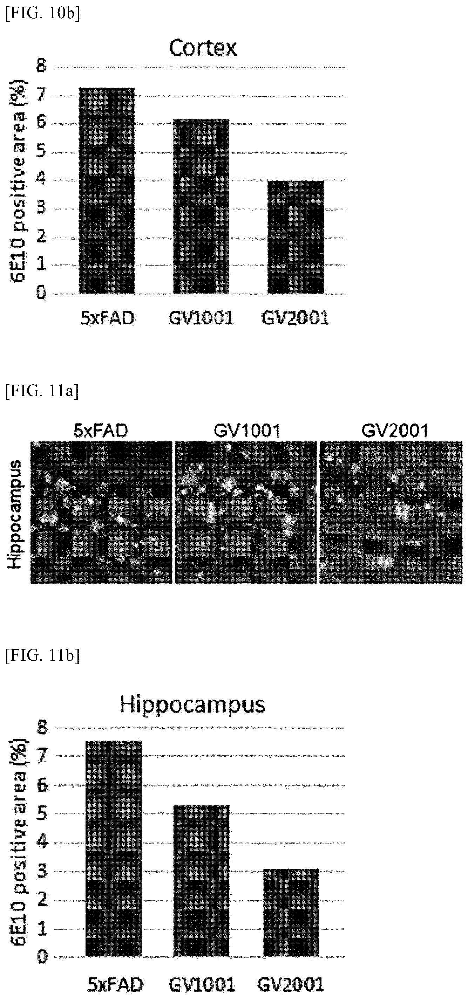

As a result of evaluating amyloid-β protein levels through 6E10 antibody staining, the GV2001 group exhibited a more pronounced decrease in amyloid-β protein levels in both the cerebral cortex and hippocampus (FIGS. 10 and 11).

### 4-5. Evaluation of Gliosis Reduction

Mouse brain tissue sections were stained with an anti-Iba-1 antibody, which stains microglia, to analyze the level of gliosis in the mouse brain.

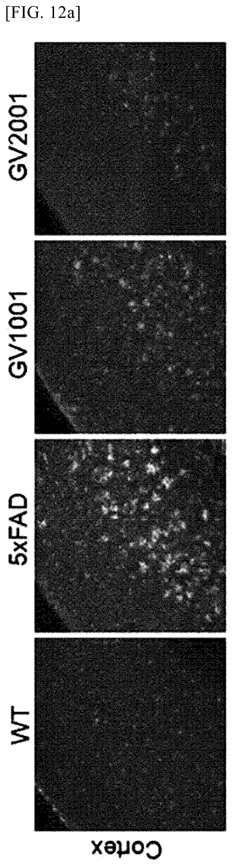

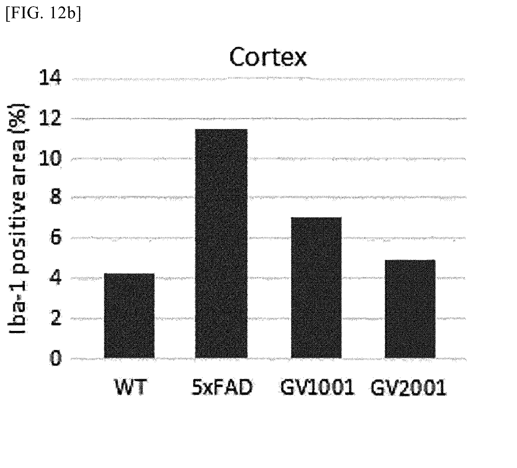

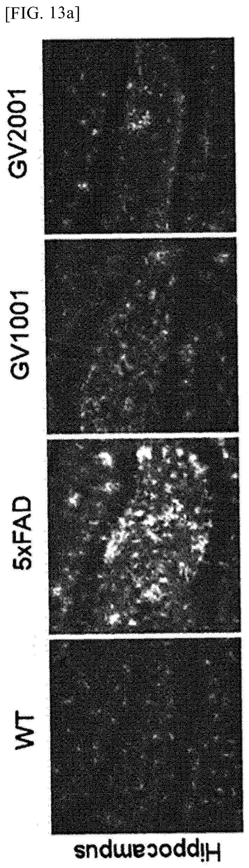

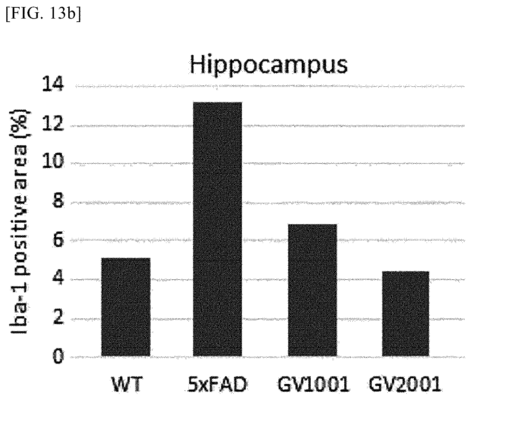

Microglia staining results showed that the GV2001 group exhibited a more pronounced decrease in gliosis levels (FIGS. 12 and 13).

### [Example 5] Evaluation of the Efficacy of GV2001 Based on an Age-Related Macular degeneration Cell Model

A cell model of age-related macular degeneration was established by treating the retinal pigment epithelial cell line ARPE-19 with hydrogen peroxide (H₂O₂) to generate reactive oxygen species.

The experimental groups were set up according to Table 4 below, and the efficacy of GV2001 was evaluated.

**[TABLE 4]**

| Group | Test article | |
|---|---|---|
| Negative control | Saline | |
| | Saline | |
| | GV2001 pre-treatment | 0.01 µM |
| | | 0.1 µM |
| | | 1.0 µM |
| H₂O₂(50 µM) treatment group | | 10 µM |
| | GV2001 post-treatment | 0.01 µM |
| | | 0.1 µM |
| | | 1.0 µM |
| | | 10 µM |
| | Metformin (1 mM) | |

| | | |
|---|---|---|
| * GV2001 pretreatment was performed by treating cells with GV2001 for 2 hours, followed by H₂O₂ treatment for 24 hours. * GV2001 post-treatment was performed by treating cells with H₂O₂ for 2 hours, followed by GV2001 treatment for 24 hours. * 1 mM metformin (Met), which has been reported to improve oxidative stress in H₂O₂-treated retinal pigment epithelial cells, was used as a positive control for GV2001. * All test articles were diluted in 0.9% saline before use. | | |

### 5-1. Evaluation of Reactive Oxygen Species Reduction

Using 2',7'-dichlorofluorescin diacetate (DCF-DA), a dye for detecting reactive oxygen species, changes in reactive oxygen species levels in retinal pigment epithelial cells (ARPE-19) treated with GV2001 were examined under a fluorescence microscope.

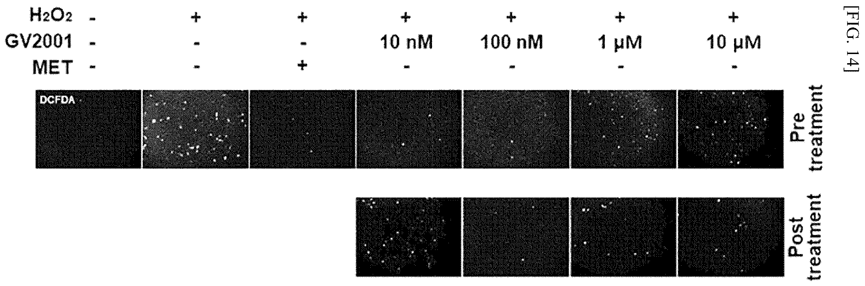

As shown in FIG. 14, oxidative stress induced by H₂O₂ increased DCF-DA fluorescence expression, which was reduced by metformin treatment, the positive control. Under oxidative stress conditions, DCF-DA fluorescence expression was decreased in the low-dose pretreatment group with GV2001 and in the high-dose post-treatment group with GV2001, demonstrating a reactive oxygen species-reducing effect.

### 5-2. Evaluation of Retinal Pigment Epithelial Cell Degeneration

The degree of epithelial-mesenchymal transition (EMT), which indicates retinal pigment epithelial cell degeneration, was evaluated by observing changes in cadherin 1, an epithelial cell marker, and vimentin, a mesenchymal cell marker, using a confocal microscope.

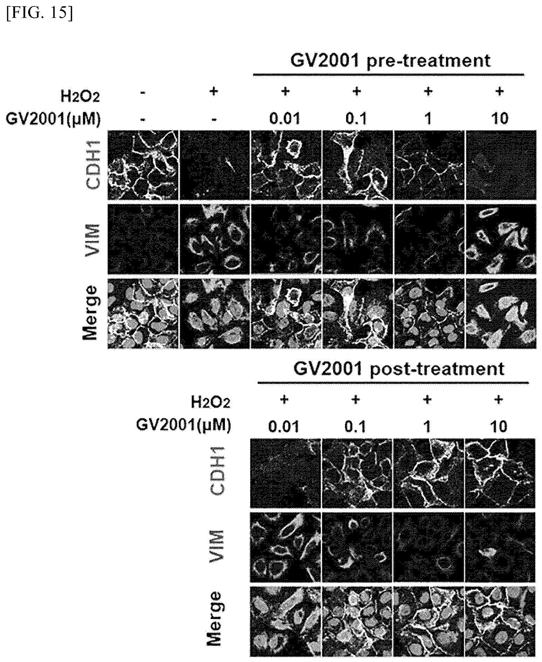

As shown in FIG. 15, oxidative stress induced by H₂O₂ decreased the expression of epithelial cell markers and increased the expression of mesenchymal cell markers, confirming the progression of EMT. Treatment with metformin, the positive control, reduced the progression of EMT. Pretreatment with GV2001 reduced the progression of EMT in the low-dose group, while post-treatment with GV2001 reduced the progression of EMT in the high-dose group.

## Claims

1. A peptide of Formula 1 below or a pharmaceutically acceptable salt thereof:

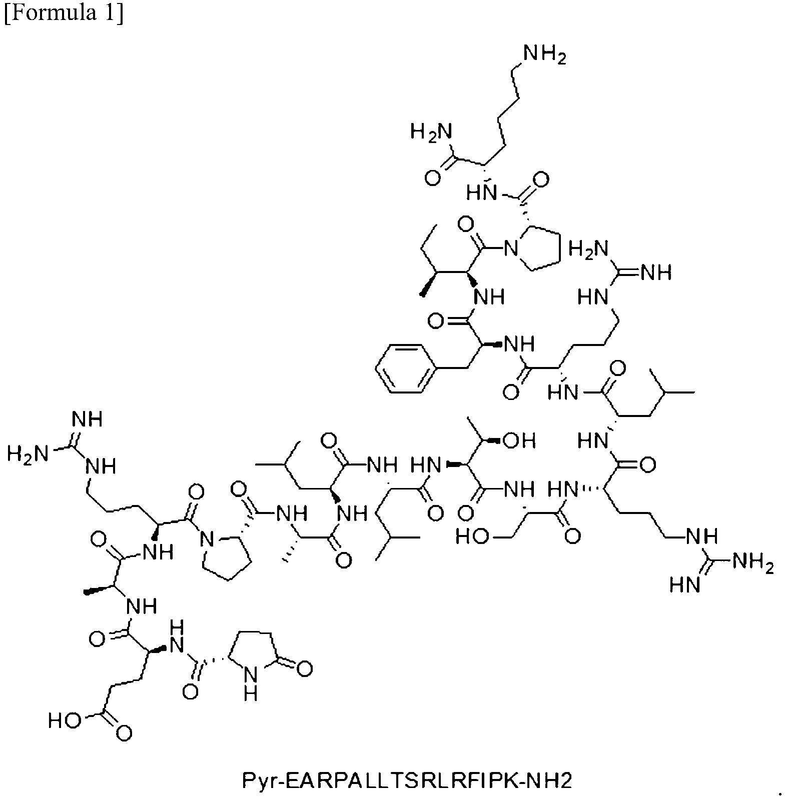

2. A pharmaceutical composition for preventing or treating amyloidosis, comprising the peptide of claim 1 or a pharmaceutically acceptable salt thereof.

3. The pharmaceutical composition according to claim 2, wherein the amyloidosis is Alzheimer's disease or cerebral amyloid angiopathy (CAA).

4. The pharmaceutical composition according to claim 2, wherein the prevention or treatment of amyloidosis is achieved by reducing the deposition of amyloid-β (Aβ).

5. A pharmaceutical composition for preventing or treating macular degeneration, comprising the peptide of claim 1 or a pharmaceutically acceptable salt thereof.

6. The pharmaceutical composition according to claim 5, wherein the macular degeneration is age-related macular degeneration (AMD).

7. The pharmaceutical composition according to claim 5, wherein the prevention or treatment of macular degeneration is achieved by reducing the degeneration of retinal pigment epithelial cells (RPE cells).

8. A health functional food for preventing or alleviating amyloidosis, comprising the peptide of Formula 1 below or a pharmaceutically acceptable salt thereof:

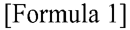

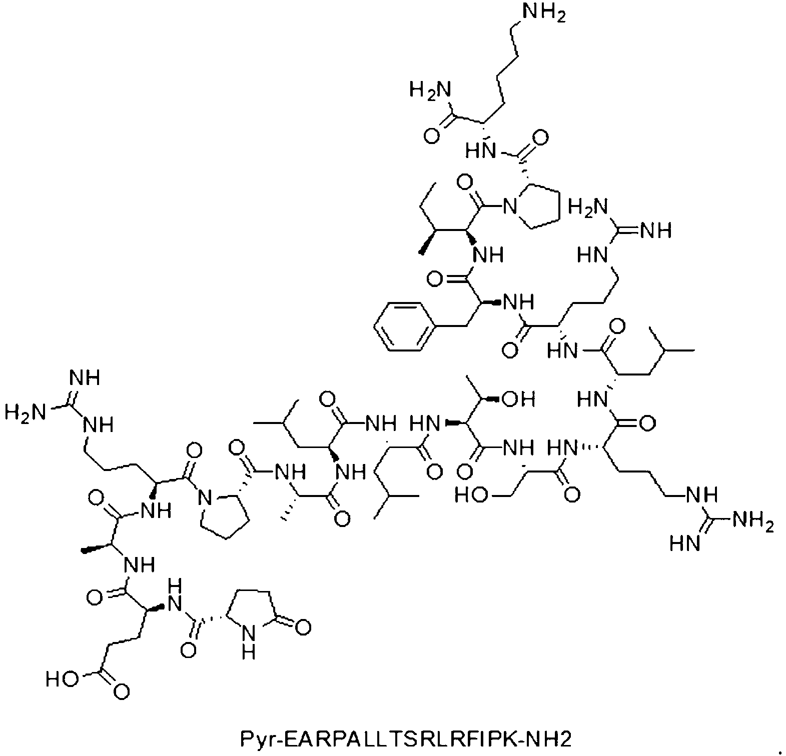

9. The health functional food according to claim 8, wherein the amyloidosis is Alzheimer's disease or cerebral amyloid angiopathy.

10. The health functional food according to claim 8, wherein the prevention or alleviation of amyloidosis is achieved by reducing the deposition of amyloid-β (Aβ).

11. A health functional food for preventing or alleviating macular degeneration, comprising a peptide of Formula 1 below or a food-acceptable salt thereof:

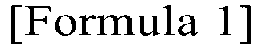

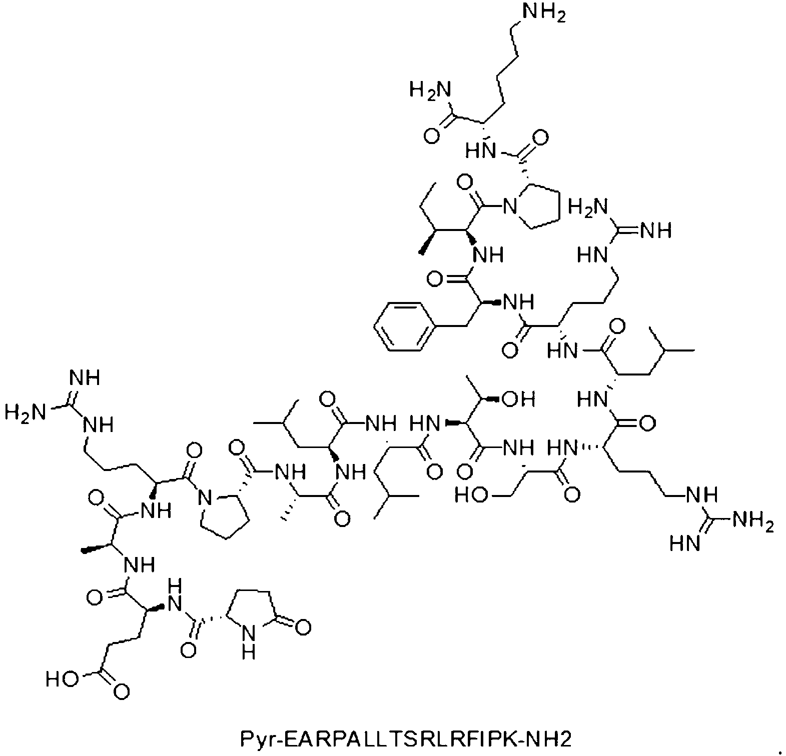

12. The health functional food according to claim 11, wherein the macular degeneration is age-related macular degeneration.

13. The health functional food according to claim 11, wherein the prevention or alleviation of macular degeneration is achieved by reducing the degeneration of retinal pigment epithelial cells.
